(19) Europäisches Patentamt
European Patent Office
Office européen des brevets

(11) **EP 1 849 470 B2**

(12) **NEW EUROPEAN PATENT SPECIFICATION**
After opposition procedure

(45) Date of publication and mention of the opposition decision:
**20.03.2024 Bulletin 2024/12**

(45) Mention of the grant of the patent:
**21.06.2017 Bulletin 2017/25**

(21) Application number: **06712292.9**

(22) Date of filing: **25.01.2006**

(51) International Patent Classification (IPC):
***A61K 31/7072*** *(2006.01)* ***A61K 31/506*** *(2006.01)*
***A61K 31/513*** *(2006.01)* ***A61K 31/7068*** *(2006.01)*
***A61P 35/00*** *(2006.01)*

(52) Cooperative Patent Classification (CPC):
(C-Sets available)
**A61K 31/506; A61K 31/7068; A61P 35/00;**
**A61P 43/00** (Cont.)

(86) International application number:
**PCT/JP2006/301097**

(87) International publication number:
**WO 2006/080327 (03.08.2006 Gazette 2006/31)**

(54) **ANTICANCER DRUG CONTAINING ALPHA, ALPHA, ALPHA-TRIFLUOROTHYMIDINE AND THYMIDINE PHOSPHORYLASE INHIBITOR**

ARZNEISTOFF GEGEN KREBS MIT ALPHA, ALPHA, ALPHA-TRIFLUORTHYMIDIN UND THYMIDIN-PHOSPHORYLASE-INHIBITOR

MÉDICAMENTS ANTICANCÉREUX CONTENANT DE LA ALPHA, ALPHA, ALPHA-TRIFLUOROTHYMIDINE ET UN INHIBITEUR DE LA THYMIDINE PHOSPHORYLASE

(84) Designated Contracting States:
**AT BE BG CH CY CZ DE DK EE ES FI FR GB GR HU IE IS IT LI LT LU LV MC NL PL PT RO SE SI SK TR**

(30) Priority: **26.01.2005 US 42059**
**06.06.2005 JP 2005165156**

(43) Date of publication of application:
**31.10.2007 Bulletin 2007/44**

(73) Proprietor: **Taiho Pharmaceutical Co., Ltd.**
**Chiyoda-ku**
**Tokyo 101-8444 (JP)**

(72) Inventors:
- **EMURA, Tomohiro**
  **Hanno-shi, Saitama, 357-8527 (JP)**
- **MITA, Akira**
  **Chiyoda-ku, Tokyo, 101-0054 (JP)**

(74) Representative: **Hartz, Nikolai et al**
**Wächtershäuser & Hartz**
**Patentanwaltspartnerschaft mbB**
**Weinstrasse 8**
**80333 München (DE)**

(56) References cited:
**EP-A1- 0 763 529** **WO-A1-96/30346**
**WO-A1-98/13045**

- **EMURA TOMOHIRO ET AL: "A novel antimetabolite, TAS-102 retains its effect on FU-related resistant cancer cells" INTERNATIONAL JOURNAL OF MOLECULAR MEDICINE, SPANDIDOS PUBLICATIONS, GR, vol. 13, no. 4, 1 January 2004 (2004-01-01), pages 545-549, XP009140488 ISSN: 1107-3756**
- **EMURA TOMOHIRO ET AL: "A novel combination antimetabolite, TAS-102, exhibits antitumor activity in FU-resistant human cancer cells through a mechanism involving FTD incorporation in DNA" INTERNATIONAL JOURNAL OF ONCOLOGY, SPANDIDOS PUBLICATIONS, GR, vol. 25, no. 3, 1 September 2004 (2004-09-01), pages 571-578, XP009140446 ISSN: 1019-6439**
- **EMURA T. ET AL.: 'An optimal dosing schedule for a novel combination antimetabolite, TAS-102, based on its intracellular metabolism and its incorporation into DNA' INTERNATIONAL JOURNAL OF MOLECULAR MEDICINE vol. 13, no. 2, 2004, pages 249 - 255, XP002997924**

EP 1 849 470 B2

- **TSUCHIYA H. ET AL.: 'Pharmacokinetic Modeling of Species-dependent Enhanced Bioavailability of Trifluorothymidine by Thymidine Phosphorylase Inhibitor' DRUG METAB. PHARMACOKIN. vol. 19, no. 3, 2004, pages 206 - 215, XP002997925**
- **Dwivedy, S. et al., "Safety and Pharmacokinetics..." Clinical Pharmacology Vol.20.2001**
- **Thomas et. at., Proceedings of the American Association of Cancer Research,43,554, #2754, 2002**

Remarks:

The file contains technical information submitted after the application was filed and not included in this specification

(52) Cooperative Patent Classification (CPC): (Cont.)

C-Sets

**A61K 31/506, A61K 2300/00;**
**A61K 31/7068, A61K 2300/00**

## Description

Technical Field

The present invention relates to a drug for the treatment of cancer (hereinafter may be referred to as "a cancer therapeutic drug") which is an anticancer agent containing α,α,α-trifluorothymidine (FTD) and a thymidine phosphorylase inhibitor (TPI) in combination, and exhibits enhanced cancer therapeutic effect.

Background Art

α,α,α-Trifluorothymidine (FTD, see the following structural formula) is a nucleoside analogue with substitution of a methyl group to trifluoromethyl group at the 5-position of thymidine, and was previously synthesized by Heidelberger, *et al.* (Non-Patent Documents 1 and 2).

[Formula 1}

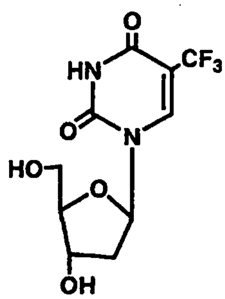

Unlike fluorouracil (FU)-based antitumor agents, which are widely used for cancer patients, FTD does not act on RNA, and is phosphorylated by intracellular thymidine kinase, thereby forming trifluorothymidine monophosphate ($F_3TMP$); i.e., a monophosphorylated form of FTD. $F_3TMP$ binds to thymidylate synthase (TS), thereby exhibiting DNA synthesis inhibitory effect (Non-Patent Documents 3 and 4). An FU-based antitumor agent has been a drug of choice in clinical practice, and its main mechanism of action is considered TS inhibition; however, recent studies have revealed that some patients are less sensitive to the FU-based antitumor agent (Non-Patent Documents 5 to 7). In contrast, FTD, which is incorporated into DNA, has been considered to exhibit an antitumor effect different from that of an FU-based antitumor agent, and thus to become a clinically useful antitumor agent overcoming the aforementioned problem. However, clinical trials of FTD performed in the 1970s have indicated a problem associated with FTD itself; i.e., intravenously administered FTD is degraded by thymidine phosphorylase (TP) *in vivo,* resulting in a very short half-life of FTD in the blood (i.e., about 12 minutes) (Non-Patent Document 8). As has also been pointed out, although intravenous administration of FTD every three hours results in tumor-shrinking effect in some patients, this administration means poses problems in that, for example, the means lacks flexibility in use and causes hematological toxicity and gastrointestinal toxicity, and the means does not necessarily contribute to survival period even in some patients showing tumor shrinkage (Non-Patent Document 9).

In order to maintain blood FTD level and to realize oral administration of FTD for improving flexibility in use, the present applicant previously found that 5-chloro-6-(1-(2-iminopyrrolidinyl)methyl)uracil hydrochloride (see the following structural formula) serves as a thymidine phosphorylase inhibitor (TPI) for suppressing degradation of FTD; and developed a cancer therapeutic drug containing FTD and the TPI in a molar ratio of 1 : 0.5 (TAS-102) (Patent Document 1 and Non-Patent Document 10).

[Formula 2]

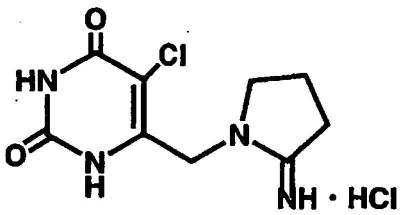

A phase I clinical trial of this combination drug was carried out in the USA. The trial, which was initiated by once-a-day oral administration, showed that blood FTD level was maintained, and this combination drug could be orally administered. However, this combination drug failed to exhibit a clinically satisfactory cancer therapeutic effect.

Non-Patent Document 1: J. Am. Chem. Soc., 84: 3597-3598, 1962
Non-Patent Document 2: J. Med. Chem., 7: 1-5, 1964
Non-Patent Document 3: Biochemistry, 33: 15086-15094, 1994
Non-Patent Document 4: Mol. Pharmacol., 1: 14-30, 1965
Non-Patent Document 5: J. Clin. Oncol., 12: 2640-2647, 1994
Non-Patent Document 6: J. Clin. Oncol., 14: 176-182, 1996
Non-Patent Document 7: J. Clin. Oncol., 21: 815-819, 2003
Non-Patent Document 8: Cancer Res., 32: 247-253, 1972
Non-Patent Document 9: Cancer Chemother. Rep., 55: 205-208, 1971
Non-Patent Document 10: International Journal of Oncology 25: 571-578, 2004
Patent Document 1: Japanese Patent No. 3088757. Emura et al., International journal of molecular medicine, vol. 13, no 2, 2004, pages 249-255, discloses a dosing schedule for TAS-102 based on its intracellular metabolism and its incorporation into DNA.

Disclosure of the Invention

Problems to be Solved by the Invention

In view of the foregoing, an object of the present invention is to provide a cancer therapeutic drug exhibiting higher efficacy.

Means for Solving the Problems

In order to achieve the aforementioned object, the present inventors have changed the dosage schedule of the above-described combination drug so that the drug is orally administered to a human twice daily. As a result, the inventors have found that, quite unexpectedly, administration of the drug at a low daily dose of 20 to 80 mg/$m^2$ (as a dose of FTD) exhibits remarkable anticancer effect, although it is that once-a-day administration of the drug requires a daily dose of 100 mg/$m^2$ (as a dose of FTD). The present invention has been accomplished on the basis of this finding.

Accordingly, the present invention provides a cancer therapeutic drug, which is a composition containing alpha, alpha, alpha-trifluorothymidine (FTD) and 5-chloro-6-(1-(2-iminopyrrolidinyl)methyl)uracil hydrochloride (hereinafter may be referred to as "TPI-1") in a molar ratio of 1 : 0.5 (hereinafter the composition may be referred to as "TAS-102") for use in the treatment of cancer in a human patient in need thereof by orally administering the drug at a dose, as dose of FTD, of 20 to 80 mg/$m^2$/day twice daily. The present disclosure also relates to the use of a composition containing $\alpha,\alpha,\alpha$-trifluorothymidine (FTD) and 5-chloro-6-(1-(2-iminopyrrolidinyl)methyl)uracil hydrochloride in a molar ratio of 1 : 0.5 for producing a cancer therapeutic drug which is orally administered to a patient in need thereof at a dose, as dose of FTD, of 20 to 80 mg/$m^2$/day twice daily to four times daily.

The present disclosure also relates to a method for treatment of cancer, characterized in that the method comprises orally administering a composition containing $\alpha,\alpha,\alpha$-trifluorothymidine (FTD) and 5-chloro-6-(1-(2-iminopyrrolidinyl)methyl)uracil hydrochloride in a molar ratio of 1 : 0.5 to a patient in need thereof at a dose, as dose of FTD, of 20 to 80 mg/$m^2$/day twice daily to four times daily.

Effects of the Invention

According to the present invention, even when the total daily dose of the cancer therapeutic drug is lower than that in the case of once-a-day administration, more excellent cancer therapeutic effect is obtained.

Brief Description of the Drawings

[Fig. 1] Fig. 1 shows comparison of therapeutic effect of a TAS-102 preparation containing FTD and TPI-1 on gastrointestinal cancer patients between the case of oral administration thrice a day and the case of oral administration once a day (PD: progressive disease, SD: stable disease, MR: minor response, PR: partial response). The vertical axis represents individual patients, whereas the horizontal axis represents the number of treatment courses. In one treatment course (a total of four weeks), a weekly dosage cycle consisting of five-day administration and two-day rest is carried out for two weeks, followed by rest for two weeks. If necessary, the rest period may be gradually prolonged in consideration of, for example, the health condition of patients and the degree of side effects or the like.

[Fig. 2] Fig. 2 shows comparison of therapeutic effect of a TAS-102 preparation containing FTD and TPI-1 on breast cancer patients in the case of oral administration twice a day (PD: progressive disease, SD: stable disease, MR: minor response, PR: partial response). The vertical axis represents individual patients, whereas the horizontal axis represents the number of treatment courses. The treatment course is the same as described above.

Best Modes for Carrying Out the Invention

The composition used in the present invention contains FTD and TPI-1 in a molar ration of 1 : 0.5. FTD (i.e., α,α,α-trifluorothymidine) is a drug which inhibits growth of cancer cells through the following mechanism: FTD is phosphorylated by intracellular thymidine kinase, thereby forming $F_3$TMP, and the thus-formed $F_3$TMP binds to thymidine synthase, thereby exhibiting DNA synthesis inhibitory effect. Meanwhile, TPI-1 is a drug which prevents inactivation of FTD due to degradation by inhibiting thymidine phosphorylase, which is a degradative enzyme for FTD.

No particular limitation is imposed on the composition, so long as it can be orally administered. The composition may be in the form of a single preparation containing both FTD and TPI-1, or combination of an FTD-containing preparation and a TPI-1-containing preparation. Examples of the form of such a preparation include tablets, coated tablets, pills, powders, granules, capsules, solutions, suspensions, and emulsions or the like. Such preparations may be formulated by use of, for example, a pharmaceutically acceptable carrier and the like through a conventional formulation method which is generally known in the art. Such a preparation may be appropriately divided and packaged so that a dose of 20 to 80 $mg/m^2$/day is administered twice daily. No particular limitation is imposed on the method for packaging a preparation, so long as the method is an established method generally known in the art. For example, a tablet may be packaged in a material used for moisture- and oxygen-impervious packaging.

Examples of carriers which may be used for forming tablets include excipients such as lactose, sucrose, sodium chloride, glucose, urea, starch, calcium carbonate, kaolin, crystalline cellulose, and silicic acid; binders such as water, ethanol, propanol, cornstarch, simple syrup, glucose solution, starch solution, gelatin solution, carboxymethylcellulose, shellac, methylcellulose, hydroxypropylcellulose, hydroxypropylmethylcellulose, potassium phosphate, and polyvinylpyrrolidone; disintegrators such as dry starch, sodium alginate, agar powder, laminaran powder, sodium hydrogencarbonate, calcium carbonate, polyoxyethylene sorbitan fatty acid esters, sodium lauryl sulfate, monoglyceride stearate, starch, and lactose; disintegration inhibitors such as sucrose, stearic acid, cacao butter, and hydrogenated oil; absorption promoters such as quaternary ammonium bases and sodium lauryl sulfate; humectants such as glycerin and starch; adsorbents such as starch, lactose, kaolin, bentonite, and colloidal silicic acid; and lubricants such as purified talc, stearic acid salts, boric acid powder, and polyethylene glycol. The tablets may be optionally coated with a conventional coating, and examples of such coated tablets include sugar-coated tablets, gelatin-coated tablets, enteric-coated tablets, film-coated tablets, double-layer tablets, and multi-layer tablets.

Examples of carriers which may be used for forming a pills include excipients such as glucose, lactose, starch, cacao butter, hydrogenated vegetable oil, kaolin, and talc; binders such as powdered acacia, powdered tragacanth, gelatin, and ethanol; and disintegrators such as laminaran and agar.

A capsule is prepared through an ordinary method by mixing the aforementioned active ingredients with any of the above-exemplified carriers, and by filling the resultant mixture into, for example, a hard gelatin capsule or a soft capsule.

An oral liquid preparation (e.g., an oral solution, a syrup, or an elixir) may be prepared through an ordinary method by use of, for example, a flavoring agent, a buffer, a stabilizer, or a smell correcting agent. Examples of the flavoring agent include sucrose, bitter orange peel, citric acid, and tartaric acid; examples of the buffer include sodium

citrate; and examples of the stabilizer include tragacanth, gum arabic, and gelatin.

If necessary, each of the aforementioned preparations may contain an additional additive such as a coloring agent, a preservative, a perfume, a seasoning agent, or a sweetener, or an additional pharmaceutical drug.

The composition of the present invention is orally administered at a dose of 20 to 80 mg/m$^2$/day (as a dose of FTD) twice daily. The daily dose is more preferably 25 to 75 mg/m$^2$/day, much more preferably 30 to 75 mg/m$^2$/day, particularly preferably 50 to 70 mg/m$^2$/day, as a dose of FTD. Dose of the composition administered to a patient in need thereof is determined on the basis of the body surface area (BSA) of the patient calculated from the patient's height and body weight. Body surface area of a patient is calculated through a conventional method which is appropriately selected in consideration of, for example, the race, sex, health condition, and symptom of the patient. Body surface area is calculated by use of, for example, any of the following calculation formulas 1 to 5, preferably the formula 1 or 2(a).

1. The Mosteller formula (see N. Engl. J. Med. 1987 Oct 22; 317 (17): 1098 (letter))

$$\text{BSA (m}^2\text{)} = ([\text{height (cm)} \times \text{weight (kg)}]/3600)^{1/2}$$

2. The DuBois and DuBois formula (see Arch. Int. Med. 1916 17: 863-71; J. Clin. Anesth. 1992; 4 (1): 4-10)

(a)

$$\text{BSA (m}^2\text{)} = 0.20247 \times \text{height (m)}^{0.725} \times \text{weight (kg)}^{0.425}$$

(b)

$$\text{BSA (m}^2\text{)} = 0.007184 \times \text{height (cm)}^{0.725} \times \text{weight (kg)}^{0.425}$$

3. The Haycock formula (see The Journal of Pediatrics 1978 93: 1: 62-66)

$$\text{BSA (m}^2\text{)} = 0.024265 \times \text{height (cm)}^{0.3964} \times \text{weight (kg)}^{0.5378}$$

4. The Gehan and George formula (see Cancer Chemother. Rep. 1970 54: 225-35)

$$\text{BSA (m}^2\text{)} = 0.0235 \times \text{height (cm)}^{0.42246} \times \text{weight (kg)}^{0.51456}$$

5. The Boyd formula (see Minneapolis: University of Minnesota Press, 1935)

$$\text{BSA (m}^2\text{)} = 0.0003207 \times \text{height (cm)}^{0.3} \times \text{weight (grams)}^{(0.7285-(0.0188\ \text{X LOG(grams)})}$$

When, for example, body surface area of a cancer patient having a height of 175 cm and a body weight of 70 kg is calculated by use of the aforementioned calculation formula 1, the body surface area is determined to be ([175 (cm) $\times$ 70 (kg)]/3600)$^{1/2}$ = 1.84 (m$^2$). When a dose of 60 mg/m$^2$/day is applied to the patient, the total daily dose is determined to be about 110 mg (i.e., 1.84 $\times$ 60 = 111 mg), and this daily dose is administered twice daily to four times daily.

In the present invention, the composition is orally administered at a dose of 20 to 80 mg/m$^2$/day (as a dose of FTD) twice daily. Preferably, the composition is administered at intervals of six hours or more.

In the present invention, a weekly dosage schedule may consist of daily administration, but preferably, the schedule consists of five-day administration and two-day rest, from the viewpoint of reduction of burden on patients. More preferably, a weekly dosage cycle consisting of five-day administration and two-day rest is carried out for two weeks, followed by rest for two weeks.

Examples of the cancer to which the cancer therapeutic drug of the present invention is applied include, but are not particularly limited to, esophageal cancer, gastric cancer, liver cancer, gallbladder/bile duct cancer, pancreatic cancer, colorectal cancer, head and neck cancer, lung cancer, breast cancer, cervical cancer, ovarian cancer, bladder

cancer, prostate cancer, testicular tumor, soft tissue and bone sarcoma, skin cancer, malignant lymphoma, leukemia, and brain tumor. Preferred are malignant solid cancers such as gastric cancer, pancreatic cancer, breast cancer, colorectal cancer, head and neck cancer, gallbladder/bile duct cancer, and lung cancer.

According to the present invention, even when the therapeutic drug is administered at a dose lower than that in the case of conventional once-a-day administration, very excellent cancer therapeutic effect is obtained. This is because, the amount of FTD incorporated into target site DNA is increased through administration twice daily. In addition, the method of the present invention facilitates control of side effects.

Examples

The present invention will next be described in more detail by way of examples.

Formulation Example 1.

| | |
|---|---|
| FTD | 20.00 mg |
| TPI-1 | 9.42 mg |
| Lactose | 70.00 mg |
| Crystalline cellulose | 3.50 mg |
| Magnesium stearate | 1.00 mg |
| Talc | 1.00 mg |
| Cornstarch | 3.50 mg |
| Hydroxypropylmethylcellulose | 25.00 mg |
| Total weight (per tablet) | 133.42 mg |

Tablets were prepared from the aforementioned formulation ratio according to an ordinary method.

Formulation Example 2.

| | |
|---|---|
| FTD | 15.00 mg |
| TPI-1 | 7.07 mg |
| Lactose | 45.00 mg |
| Carboxymethylcellulose | 5.00 mg |
| Magnesium stearate | 2.00 mg |
| Titanium oxide | 0.50 mg |
| Hydroxypropylmethylcellulose | 1.00 mg |
| Polyethylene glycol 4000 | 0.50 mg |
| Total weight (per tablet) | 85.07 mg |

Tablets were prepared from the aforementioned formulation ratio according to an ordinary method.

Formulation Example 3.

| | |
|---|---|
| FTD | 30.00 mg |
| TPI-1 | 14.13 mg |
| Lactose | 85.00 mg |
| Cornstarch | 100.00 mg |
| Hydroxypropylcellulose | 2.50 mg |
| Total weight (per package) | 231.63 mg |

Granules were prepared from the aforementioned formulation ratio according to an ordinary method.

Formulation Example 4.

| | |
|---|---|
| FTD | 10.00 mg |
| TPI-1 | 4.71 mg |
| Lactose | 24.00 mg |
| Crystalline cellulose | 12.50 mg |

(continued)

| | |
|---|---|
| Magnesium stearate | 1.00 mg |
| Total weight (per capsule) | 52.21 mg |

Capsules were prepared from the aforementioned formulation ratio according to an ordinary method.

Example 1. (Reference Example)

Therapeutic effect of TAS-102 was studied by orally administering TAS-102 to cancer patients at a dose of 100 mg/m$^2$ (as a dose of FTD) once a day (trial 1), or at a dose of 70 mg/m$^2$ (as a dose of FTD) thrice a day (trial 2).

These trials were performed in patients with gastrointestinal cancer for which standard therapy is ineffective or no curative therapy exists, for the main purpose of evaluating safety of TAS-102. The trials correspond to a phase I clinical trial for determining the recommended dose (RD) of TAS-102 at which the drug can be safely administered without causing problematic side effects in phase II clinical trials carried out in each of different cancer types. If possible, therapeutic effect of the drug on tumor is evaluated through the trials. For evaluation of therapeutic effect on tumor, the tumor-shrinking effect of the drug was determined on the basis of comprehensive evaluation of target lesions (i.e., lesions having a size equal to or greater than a measurable size corresponding to slice thickness in CT or a similar technique) and non-target lesions (i.e., all lesions other than the target lesions) with reference to the RECIST evaluation method (Journal of the National Cancer Institute, 2000, Vol. 92, No. 3, 205-216). In the trials, "PR (partial response)" refers to the case where the sum of the major axis lengths of target lesions is reduced by 30% or more as compared with that before administration of the drug, and the effect of the drug is maintained for a predetermined period of time (generally four weeks) during which no exacerbation of non-target lesions is observed. "PD (progressive disease)" refers to the case where the sum of the major axis lengths of target lesions is increased by 20% or more as compared with the minimum sum of the major axis lengths as recorded after initiation of the trials, or the case where existing non-target lesions are apparently exacerbated or new lesions are observed. "SD (stable disease)" refers to the case where tumor shrinkage is not enough to be regarded as "PR," whereas tumor progression is not enough to be regarded as "PD"; i.e., progression of tumor is stopped, and no exacerbation of tumor is observed. "MR (minor response)" refers to the case where tumor-shrinking effect is lower as compared with that of "PR" (i.e., reduction in the sum of the lesion major axis lengths is less than 30%), but is maintained at a comparable level (i.e., reduction in the sum of the lesion major axis lengths is about 15%), or the case where therapeutic effect corresponding to "PR" is observed temporarily.

The results are shown in Fig. 1. In Fig. 1, "Trial 1" corresponds to administration of a TAS-102 preparation (tablets) at a dose of 100 mg/m$^2$ (as a dose of FTD) once a day (five-day administration and two-day rest per week). The data show that the drug administration was effective (i.e., stable disease without exacerbation of tumor) for two of six patients (33%). "Trial 2" corresponds to administration of a TAS-102 preparation (tablets) at a dose of 70 mg/m$^2$ (as a dose of FTD) thrice daily (five-day administration and two-day rest per week). The data show that the drug administration was effective for four of six patients (67%). Specifically, in the four patients, progression of tumor was stopped, and no exacerbation of tumor was observed; and, in one of the four patients, tumor shrinkage was also observed. Thus, these data suggest that administration of TAS-102 in a divided manner is an effective administration method for patients with gastrointestinal cancer for which standard therapy is ineffective or no curative therapy exists.

Example 2.

Similar to the case of Example 1, phase I clinical trials were performed in breast cancer patients.

Therapeutic effect of TAS-102 was studied by orally administering TAS-102 to patients with breast cancer for which standard therapy is ineffective or no curative therapy exists at a dose of 60 mg/m$^2$/day (as a dose of FTD) twice a day (trial 3), or at a dose of 50 mg/m$^2$/day (as a dose of FTD) twice a day (trial 4).

The results are shown in Fig. 2. "Trial 3" corresponds to administration of a TAS-102 preparation (tablets) at a dose of 60 mg/m$^2$ (as a dose of FTD) twice daily (five-day administration and two-day rest per week). The data show that the drug administration was effective for five of seven patients (71%). "Trial 4" corresponds to administration of a TAS-102 preparation (tablets) at a dose of 50 mg/m$^2$ (as a dose of FTD) twice daily (five-day administration and two-day rest per week). The data show that the drug administration was effective for seven of nine patients (78%). Specifically, in most patients, progression of tumor was stopped, and no exacerbation of tumor was observed. In a plurality of patients, SD was continued for six months or more, and, in one patient, SD was continued for one year or more. In breast cancer therapy, a therapeutic method which can be continuously carried out for six courses (about six months) or more is considered to have high clinical utility. Thus, these data suggest that, similar to the case of Example 1, administration of TAS-102 in a divided manner is an effective administration method for patients with breast cancer for which standard therapy is ineffective or no curative therapy exists.

## Claims

1. A cancer therapeutic drug, which is a composition comprising α,α,α-trifluorothymidine (FTD) and 5-chloro-6-(1-(2-iminopyrrolidinyl)methyl)uracil hydrochloride in a molar ratio of 1 : 0.5, for use in the treatment of cancer in a human patient in need thereof by orally administering the drug at a dose, as a dose of FTD, of 20 to 80 mg/m$^2$/day twice daily.

2. The cancer therapeutic drug according to claim 1, wherein the drug is orally administered to a patient in need thereof at a dose, as a dose of FTD, of 25 to 75 mg/m$^2$/day administered twice daily.

3. The cancer therapeutic drug according to claim 1, wherein the drug is administered twice daily on a week-based schedule which includes five-day administration and two-day rest.

4. The cancer therapeutic drug according to claim 1, wherein the drug is administered in such a manner that a weekly dosage cycle consisting of five-day administration and two-day rest is carried out for two weeks, followed by rest for two weeks.

5. The cancer therapeutic drug according to claim 1, wherein the drug is administered to a patient in need thereof at a dose, as a dose of FTD, of 50 to 70 mg/m$^2$/day.

6. The cancer therapeutic drug according to any one of claims 1 to 5, wherein the cancer to which the cancer therapeutic drug is applied includes esophageal cancer, gastric cancer, liver cancer, gallbladder/bile duct cancer, pancreatic cancer, colorectal cancer, head and neck cancer, lung cancer, breast cancer, cervical cancer, ovarian cancer, bladder cancer, prostate cancer, testicular tumor, soft tissue and bone sarcoma, skin cancer, malignant lymphoma, leukemia, and brain tumor.

7. The cancer therapeutic drug according to any one of claims 1 to 6, wherein the cancer to which the cancer therapeutic drug is applied includes malignant solid cancers selected from gastric cancer, pancreatic cancer, breast cancer, colorectal cancer, head and neck cancer, gallbladder/bile duct cancer, and lung cancer.

8. The cancer therapeutic drug according to any one of claims 1 to 7, wherein the dose of the composition administered to a patient in need thereof is determined on the basis of the body surface area of the patient, which body surface area is calculated byuse of any of the following calculation formulas 1 to 5:

1. The Mosteller formula (see N. Engl. J. Med. 1987 Oct 22; 317 (17): 1098 (letter))

$$BSA\ (m^2) = ([\text{height (cm)} \times \text{weight (kg)}]/3600)^{1/2}$$

2. The DuBois and DuBois formula (see Arch. Int. Med. 1916 17: 863-71; J. Clin. Anesth. 1992; 4 (1): 4-10 )

(a)

$$BSA\ (m^2) = 0.20247 \times \text{height (m)}^{0.725} \times \text{weight (kg)}^{0.425}$$

(b)

$$BSA\ (m^2) = 0.007184 \times \text{height (cm)}^{0.725} \times \text{weight (kg)}^{0.425}$$

3. The Haycock formula (see The Journal of Pediatrics 1978 93: 1: 62-66)

$$BSA\ (m^2) = 0.024265 \times \text{height (cm)}^{0.3964} \times \text{weight (kg)}^{0.5378}$$

4. The Gehan and George formula (see Cancer Chemother. Rep. 1970 54: 225-35 )

$$BSA\ (m^2) = 0.0235 \times height\ (cm)^{0.42246} \times weight\ (kg)^{0.51456}$$

5. The Boyd formula (see Minneapolis: University of Minnesota Press, 1935)

$$BSA\ (m^2) = 0.0003207 \times height\ (cm)^{0.3} \times weight\ (grams)^{(0.7285-(0.0188 \times LOG(grams))}$$

## Patentansprüche

**1.** Therapeutisches Krebsmedikament, welches eine Zusammensetzung ist, die α,α,α-Trifluorthymidin (FTD) und 5-Chlor-6-(1-(2-iminopyrrolidinyl)methyl)uracil-hydrochlorid in einem molaren Verhältnis von 1:0,5 umfasst, zur Verwendung bei der Behandlung von Krebs in einem menschlichen Patienten, der dessen bedarf, durch orale Verabreichung des Medikaments in einer Dosis, als Dosis von FTD, von 20 bis 80 $mg/m^2$/Tag, zweimal täglich.

**2.** Therapeutisches Krebsmedikament zur Verwendung nach Anspruch 1, wobei das Medikament einem Patienten, der dessen bedarf, in einer Dosis, als Dosis von FTD, von 25 bis 75 $mg/m^2$/Tag, zweimal täglich verabreicht wird.

**3.** Therapeutisches Krebsmedikament zur Verwendung nach Anspruch 1, wobei das Medikament zweimal täglich nach einem auf einer Woche basierenden Plan, der eine Verabreichung für 5 Tage und eine zweitägige Pause einschließt, verabreicht wird.

**4.** Therapeutisches Krebsmedikament zur Verwendung nach Anspruch 1, wobei das Medikament auf eine solche Weise verabreicht wird, dass ein wöchentlicher Dosiszyklus, der aus einer Verabreichung für 5 Tage und einer zweitägigen Pause besteht, für zwei Wochen durchgeführt wird, gefolgt von einer zweiwöchigen Pause.

**5.** Therapeutisches Krebsmedikament zur Verwendung nach Anspruch 1, wobei das Medikament einem Patienten, der dessen bedarf, mit einer Dosis, als Dosis von FTD, von 50 bis 70 $mg/m^2$/Tag verabreicht wird.

**6.** Therapeutisches Krebsmedikament zur Verwendung nach einem der Ansprüche 1 bis 5, wobei der Krebs, an dem das therapeutische Krebsmedikament angewendet wird, Speiseröhrenkrebs, Magenkrebs, Leberkrebs, Gallenblasen-/Gallengangkrebs, Bauchspeicheldrüsenkrebs, Dickdarmkrebs, Kopf-Hals-Karzinom, Lungenkrebs, Brustkrebs, Gebärmutterhalskrebs, Ovarialkarzinom, Blasenkrebs, Prostatakrebs, Hodenkrebs, Weichteil- und Knochensarkome, Hautkrebs, malignes Lymphom, Leukämie und Hirntumor einschließt.

**7.** Therapeutisches Krebsmedikament zur Verwendung nach einem der Ansprüche 1 bis 6, wobei der Krebs, an dem das therapeutische Krebsmedikament angewendet wird, maligne solide Tumore einschließt, ausgewählt aus Magenkrebs, Bauchspeicheldrüsenkrebs, Brustkrebs, Dickdarmkrebs, Kopf-Hals-Karzinom, Gallenblasen-/Gallengangkrebs und Lungenkrebs.

**8.** Therapeutisches Krebsmedikament zur Verwendung nach einem der Ansprüche 1 bis 7, wobei die Dosis der Zusammensetzung, die an den Patienten, der dessen bedarf, verabreicht wird, auf Basis der Körperoberfläche des Patienten bestimmt wird, wobei die Körperoberfläche durch Verwenden einer beliebigen der folgenden Berechnungsformeln 1 bis 5 berechnet wird:

1. Die Mosteller-Formel (siehe N. Engl. J. Med. 1987 Oct 22; 317 (17): 1098 (Letter))

$$\text{Körperoberfläche}\ (m^2) = ([\text{Größe}\ (cm) \times \text{Gewicht}\ (kg)]/3600)^{1/2}$$

2. Die DuBois- und DuBois-Formel (siehe Arch. Int. Med. 1916 17: 863-71; J. Clin. Anesth. 1992; 4 (1): 4-10)

(a)

$$\text{Körperoberfläche}\ (m^2) = 0{,}20247 \times \text{Größe}\ (m)^{0{,}725} \times \text{Gewicht}\ (kg)^{0{,}425}$$

(b)

$$\text{Körperoberfläche } (m^2) = 0{,}007184 \times \text{Größe (cm)}^{0{,}725} \times \text{Gewicht (kg)}^{0{,}425}$$

3. Die Haycock-Formel (siehe The Journal of Pediatrics 1978 93: 1: 62-66)

$$\text{Körperoberfläche } (m^2) = 0{,}024265 \times \text{Größe (cm)}^{0{,}3964} \times \text{Gewicht (kg)}^{0{,}5378}$$

4. Die Gehan and George-Formel (siehe Cancer Chemother. Rep. 1970 54: 225-35)

$$\text{Körperoberfläche } (m^2) = 0{,}0235 \times \text{Größe (cm)}^{0{,}42246} \times \text{Gewicht (kg)}^{0{,}51456}$$

5. Die Boyd-Formel (siehe Minneapolis: University of Minnesota Press, 1935)

$$\text{Körperoberfläche } (m^2) = 0{,}0003207 \times \text{Größe (cm)}^{0{,}3} \times \text{Gewicht (Gramm)}^{(0{,}7285-(0{,}0188 \text{ X LOG(Gramm)})}$$

**Revendications**

**1.** Médicament thérapeutique anticancéreux, qui est une composition comprenant de l'$\alpha,\alpha,\alpha$-trifluorothymidine (FTD) et du chlorhydrate de 5-chloro-6-(1-(2-iminopyrrolidinyl)méthyl)uracile en un rapport molaire de 1/0,5, pour une utilisation dans le traitement d'un cancer chez un patient humain en ayant besoin, par administration par voie orale du médicament à une dose, ramenée à la dose de FTD, de 20 à 80 mg/$m^2$/jour deux fois par jour.

**2.** Médicament thérapeutique anticancéreux pour une utilisation selon la revendication 1, lequel médicament est administré par voie orale à un patient en ayant besoin à une dose, ramenée à la dose de FTD, de 25 à 75 mg/$m^2$/jour administrée deux fois par jour.

**3.** Médicament thérapeutique anticancéreux pour une utilisation selon la revendication 1, lequel médicament est administré deux fois par jour selon un schéma hebdomadaire qui comprend cinq jours d'administration et deux jours de repos.

**4.** Médicament thérapeutique anticancéreux pour une utilisation selon la revendication 1, lequel médicament est administré de façon qu'un cycle d'administration hebdomadaire consistant en cinq jours d'administration et deux jours de repos soit effectué pendant deux semaines, suivies de deux semaines de repos.

**5.** Médicament thérapeutique anticancéreux pour une utilisation selon la revendication 1, lequel médicament est administré à un patient en ayant besoin à une dose, ramenée à la dose de FTD, de 50 à 70 mg/$m^2$/jour.

**6.** Médicament thérapeutique anticancéreux pour une utilisation selon l'une quelconque des revendications 1 à 5, dans lequel le cancer auquel le médicament thérapeutique anticancéreux est appliqué comprend un cancer de l'œsophage, un cancer gastrique, un cancer du foie, un cancer de la vésicule biliaire/des voies biliaires, un cancer pancréatique, un cancer colorectal, un cancer de la tête et du cou, un cancer du poumon, un cancer du sein, un cancer du col, un cancer ovarien, un cancer de la vessie, un cancer de la prostate, une tumeur testiculaire, un sarcome des tissus mous et des os, un cancer de la peau, un lymphome malin, une leucémie, et une tumeur au cerveau.

**7.** Médicament thérapeutique anticancéreux pour une utilisation selon l'une quelconque des revendications 1 à 6, dans lequel le cancer auquel le médicament thérapeutique anticancéreux est appliqué comprend les cancers solides malins choisis parmi un cancer gastrique, un cancer pancréatique, un cancer du sein, un cancer colorectal, un cancer de la tête et du cou, un cancer de la vésicule biliaire/des voies biliaires, et un cancer du poumon.

**8.** Médicament thérapeutique anticancéreux pour une utilisation selon l'une quelconque des revendications 1 à 7, dans

lequel la dose de la composition administrée à un patient en ayant besoin est déterminée sur la base de la surface corporelle du patient, laquelle surface corporelle est calculée par l'utilisation de l'une quelconque des formules de calcul 1 à 5 suivantes :

1. la formule de Mosteller (voir N. Engl. J. Med. du 22 octobre 1987 ; 317 (17) : 1098 (lettre))

$$\text{surface corporelle (BSA) } (m^2) = ([\text{hauteur (cm) x poids (kg)}] / 3600)^{1/2}$$

2. la formule de DuBois et DuBois (voir Arch. Int. Med. 1916 17 : 863-71 ; J. Clin. Anesth. 1992 ; 4 (1) : 4-10)

(a)

$$\text{(a) surface corporelle (BSA) } (m^2) = 0{,}20247 \text{ x hauteur (m)}^{0{,}725} \text{ x poids (kg)}^{0{,}425}$$

(b)

$$\text{(b) surface corporelle (BSA) } (m^2) = 0{,}007184 \text{ x hauteur (cm)}^{0{,}725} \text{ x poids (kg)}^{0{,}425}$$

3. la formule de Haycock (voir The Journal of Pediatrics 1978 93 : 1 : 62-66)

$$\text{surface corporelle (BSA) } (m^2) = 0{,}024265 \text{ x hauteur (cm)}^{0{,}3964} \text{ x poids (kg)}^{0{,}5378}$$

4. la formule de Gehan et George (voir Cancer Chemother. Rep. 1970 54 : 225-35)

$$\text{surface corporelle (BSA) } (m^2) = 0{,}0235 \text{ x hauteur (cm)}^{0{,}42246} \text{ x poids (kg)}^{0{,}51456}$$

5. la formule de Boyd (voir Minneapolis : University of Minnesota Press, 1935)

$$\text{surface corporelle (BSA) } (m^2) = 0{,}0003207 \text{ x hauteur (cm)}^{0{,}3} \text{ x poids (grammes)}^{(0{,}7285-(0{,}0188\text{xLOG(grammes)})}.$$

Fig. 1

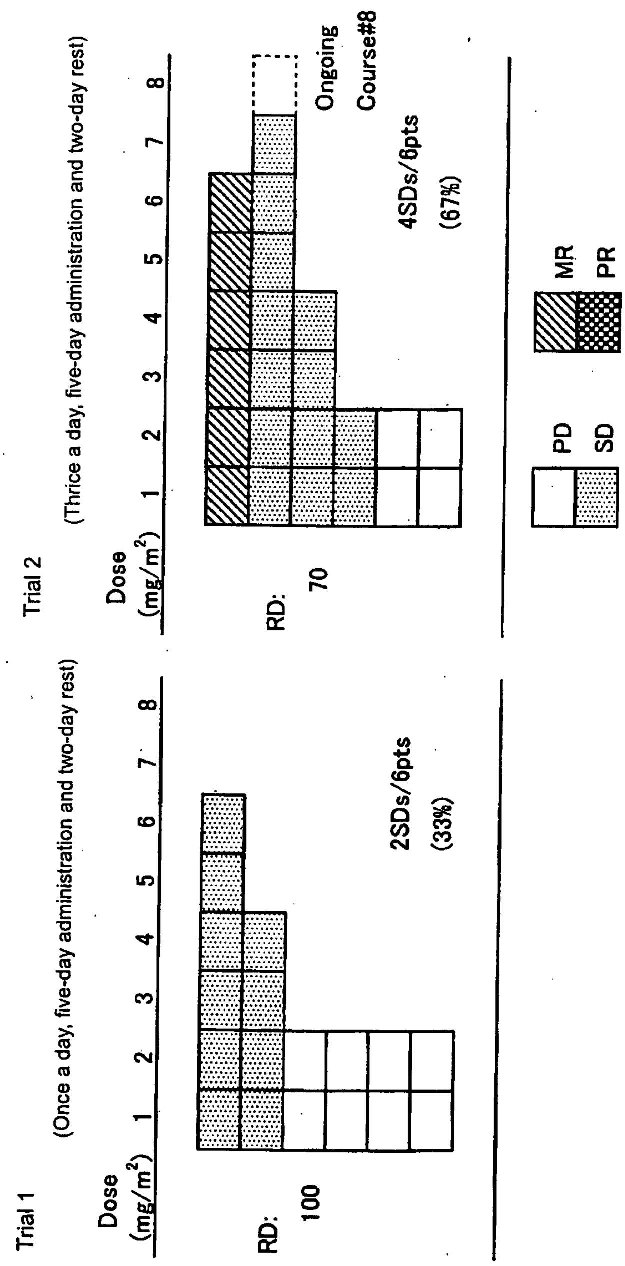
Trial 1
(Once a day, five-day administration and two-day rest)
Dose (mg/m2)
1 2 3 4 5 6 7 8
RD: 100
2SDs/6pts
(33%)
Trial 2
(Thrice a day, five-day administration and two-day rest)
Dose (mg/m2)
1 2 3 4 5 6 7 8
RD: 70
Ongoing Course#8
4SDs/6pts
(67%)
PD
SD
MR
PR

Fig. 2

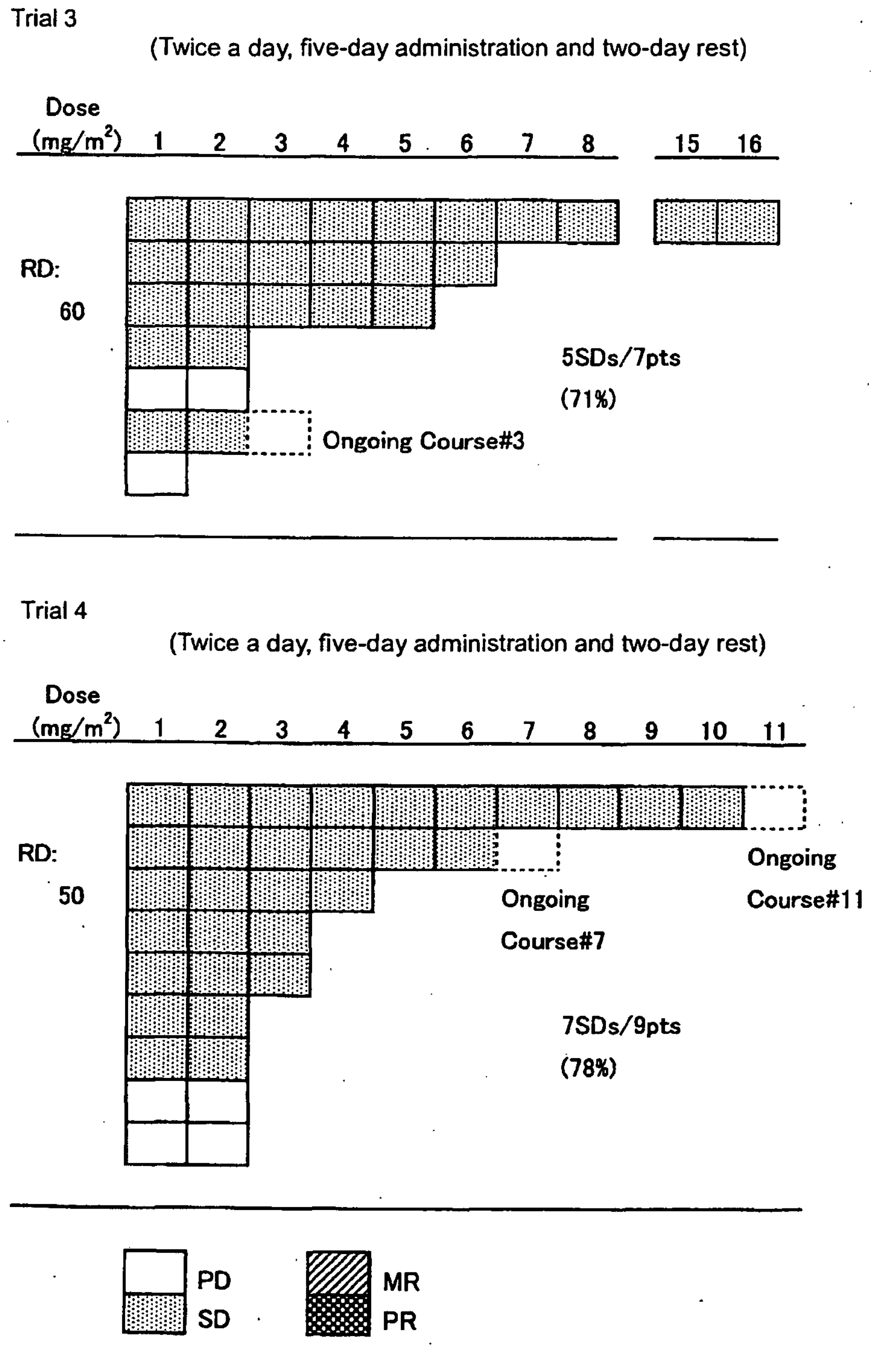
Trial 3
(Twice a day, five-day administration and two-day rest)
Dose (mg/m2)
1 2 3 4 5 6 7 8 15 16
RD: 60
5SDs/7pts
(71%)
Ongoing Course#3
Trial 4
(Twice a day, five-day administration and two-day rest)
Dose (mg/m2)
1 2 3 4 5 6 7 8 9 10 11
RD: 50
Ongoing Course#7
Ongoing Course#11
7SDs/9pts
(78%)
PD
SD
MR
PR

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3088757 B


### Non-patent literature cited in the description


- *J. Am. Chem. Soc.,* 1962, vol. 84, 3597-3598
- *J. Med. Chem.,* 1964, vol. 7, 1-5
- *Biochemistry,* 1994, vol. 33, 15086-15094
- *Mol. Pharmacol.,* 1965, vol. 1, 14-30
- *J. Clin. Oncol.,* 1994, vol. 12, 2640-2647
- *J. Clin. Oncol,* 1996, vol. 14, 176-182
- *J. Clin. Oncol.,* 2003, vol. 21, 815-819
- *Cancer Res.,* 1972, vol. 32, 247-253
- *Cancer Chemother. Rep,* 1971, vol. 55, 205-208
- *International Journal of Oncology,* 2004, vol. 25, 571-578
- **EMURA et al.** *International journal of molecular medicine,* 2004, vol. 13 (2), 249-255
- *N. Engl. J. Med.,* 22 October 1987, vol. 317 (17), 1098
- *Arch. Int. Med.,* 1916, vol. 17, 863-71
- *J. Clin. Anesth.,* 1992, vol. 4 (1), 4-10
- *The Journal of Pediatrics,* 1978, vol. 93 (1), 62-66
- *Cancer Chemother. Rep.,* 1970, vol. 54, 225-35
- *Journal of the National Cancer Institute,* 2000, vol. 92 (3), 205-216